# DEMANDE DE BREVET EUROPEEN

(11) **EP 4 674 977 A1**
(43) Date de publication de la demande: **07.01.2026**
(21) Numéro de dépôt: 25180944.8
(22) Date de dépôt: 05.06.2025
(51) Int. Cl.: C12Q 1/6806

(54) **PROCÉDÉ D' OBTENTION D'UN ÉCHANTILLON DE MATRICE ALIMENTAIRE FLUIDIFIÉ ET UTILISATION DE L' ÉCHANTILLON OBTENU POUR LA DÉTECTION DE BACTÉRIES**

(30) Priorité: 03.07.2024 FR 2407271
(71) Demandeur: Commissariat à l'Energie Atomique et aux Energies Alternatives, 75015 Paris (FR)
(72) Inventeur: BOURDAT, Anne-Gaëlle, 38054 Grenoble cedex 09 (FR); BENCHETRIT, Hugo, 38054 Grenoble cedex 09 (FR)
(74) Mandataire: INNOV-GROUP

(57) **Abrégé**

L'invention concerne un procédé d'obtention d'un échantillon (S3) de matrice alimentaire, ce procédé consistant à réaliser un mélange d'un prélèvement (S1) de ladite matrice alimentaire avec une solution (S2) comprenant un composé tensioactif de type Ethoxylate d'alcool secondaire, ledit procédé comportant également une étape de chauffage dudit échantillon (S3) obtenu, ou de chauffage préalable avant mélange de la solution (S2) contenant ledit composé tensioactif et du prélèvement (S1) de ladite matrice alimentaire, à une température adaptée pour éviter toute dégradation d'espèces biologiques (E) cibles dans l'échantillon.

## Description

### Domaine technique de l'invention

La présente invention se rapporte à un procédé d'obtention d'un échantillon de matrice alimentaire fluidifié et à l'utilisation de l'échantillon obtenu pour la détection de bactéries.

### Etat de la technique

La détection de la présence de bactéries pathogènes dans des matrices alimentaires complexes et grasses, telles que le lait, la viande, le poisson et le chocolat, constitue un défi majeur dans l'industrie alimentaire. Des pertes importantes de productions alimentaires ont en effet été constatées récemment, suite à la détection de la présence de bactéries pathogènes.

Dans le domaine de l'agroalimentaire, les analyses microbiologiques restent indispensables pour la maîtrise de la sécurité sanitaire des aliments. Ces analyses sont fiables parce qu'elles reposent sur des méthodes d'essais éprouvées.

Or, deux types de méthodes existent sur le marché : des méthodes de référence (normalisées), reposant principalement sur la culture de bactéries, ou des méthodes disponibles sous la forme de kits commerciaux (culture ou culture + méthode moléculaire ou culture + test immunologique).

Certaines méthodes de détection de bactéries pathogènes connues se concentrent généralement sur des étapes d'enrichissement en bactéries par culture, de lyse des bactéries, suivies d'une détection biomoléculaire de l'ADN des pathogènes, notamment par qPCR et LAMP (amplification isotherme). Ces solutions impliquent souvent un prélèvement sur site, suivi d'une analyse externalisée vers des laboratoires de services ou en interne dans un laboratoire sur site, rendant le processus long. De plus, ces méthodes connues ne permettent souvent pas d'engager un volume suffisamment important pour assurer une analyse fiable, sans un enrichissement préalable de l'échantillon. Or cette phase d'enrichissement peut durer plusieurs heures pour détecter les principales bactéries pathogènes telles que *Salmonella, Listeria,* et *E.coli.* De manière générale, les méthodes d'analyse suffisamment sensibles et rapides sont donc actuellement impossibles à déployer sur le terrain.

La publication référencée « Mayrl E, Roeder B, Mester P, Wagner M, Rossmanith P. Broad range evaluation of the matrix solubilization (matrix lysis) strategy for direct enumeration of foodborne pathogens by nucleic acids technologies. J Food Prot. 2009 Jun;72(6):1225-33. doi: 10.4315/0362-028x-72.6.1225. PMID: 19610333 » décrit un principe de détection de bactéries dans une matrice alimentaire (poisson, lait, viande).

La publication référencée ci-dessous décrit un procédé de détection de bactéries dans une matrice alimentaire telle que lait, poulet, viande.
*Hui Peng, Leora A. Shelef, Automated simultaneous detection of low levels of listeriae and salmonellae in foods,*
*International Journal of Food Microbiology,*
Volume 63, Issue 3, 2001, Pages 225-233, ISSN 0168-1605, https://doi.org/10.1016/S0168-1605(00)00418-9. (https://www.sciencedirect.com/science/article/pii/S0168160500004189*)*

La demande de brevet US2010/184210A1 décrit une méthode pour isoler des cellules dans un échantillon complexe.

Le but de l'invention est de proposer un procédé d'obtention d'un échantillon de matrice alimentaire fluidifié, permettant in fine une analyse fiable, rapide et robuste dudit échantillon, permise notamment par un engagement en volume important, sans enrichissement préalable de l'échantillon.

### Exposé de l'invention

Ce but est atteint par un procédé de préparation d'un échantillon de matrice alimentaire, caractérisé en ce qu'il consiste à réaliser un mélange d'un prélèvement (S1) de ladite matrice alimentaire avec une solution comprenant un composé tensioactif de type Ethoxylate d'alcool secondaire, ledit procédé comportant également une étape de chauffage dudit échantillon obtenu, ou de chauffage préalable avant mélange de la solution contenant ledit composé tensioactif et du prélèvement de ladite matrice alimentaire, à une température adaptée pour éviter toute dégradation d'espèces biologiques cibles dans l'échantillon, ledit composé tensioactif étant ajouté au prélèvement de matrice alimentaire de manière à ne pas dépasser 25% en concentration dans l'échantillon obtenu, ledit procédé comportant une étape de filtration dudit échantillon obtenu, mise en œuvre à travers un filtre en vue d'isoler lesdites espèces biologiques cibles.

Selon une particularité, le prélèvement de matrice alimentaire et la solution contenant le composé tensioactif sont chauffés séparément à une température comprise entre 35°C et 50°C.

Selon une autre particularité, le procédé comporte une étape de lyse desdites espèces biologiques isolées, mise en œuvre en vue de libérer les molécules d'ADN.

Selon une autre particularité, le procédé comporte une étape d'élution des molécules d'ADN libérées à l'aide d'un tampon d'élution est mise en œuvre après ladite état de lyse.

Selon une autre particularité, le tampon d'élution est un réactif d'amplification.

### Brève description des figures

D'autres caractéristiques et avantages vont apparaître dans la description détaillée qui suit faite en regard des dessins annexés dans lesquels :
- La figure 1 illustre les différentes étapes du procédé de l'invention ;
- La figure 2 montre un exemple de composant microfluidique pouvant être employé pour la mise en œuvre du procédé de l'invention ;
- La figure 3 montre un diagramme illustrant le suivi de bactéries dans une matrice alimentaire de type lait, lors de sa préparation et de son analyse ;
- Les figures 4A à 4C montrent des diagrammes de résultats obtenus pour une matrice alimentaire de type steak haché.

### Description détaillée d'au moins un mode de réalisation

L'invention s'applique à la préparation d'un échantillon de matrice alimentaire, en vue de pouvoir analyser cet échantillon et d'y détecter la présence de bactéries pathogènes. La matrice alimentaire est par exemple constituée de produits laitiers (lait, fromage) ou d'autres produits tels que viande, poisson, chocolat. La matrice alimentaire est avantageusement sous forme liquide ou liquéfiée (c'est-à-dire solide broyé/désagrégé dans un liquide - exemple : viande, poisson broyé dans un milieu de culture au 1/10 ou 1/4) ou légèrement pâteuse. Elle est avantageusement grasse et protéinée.

De manière non limitative, les bactéries pathogènes classiques à détecter sont par exemple connues sous le nom *Salmonella, Listeria,* et *E.coli, Cronobacter, Campylobacter, Bacillus cereus, ou des levures et moisissure.* D'autres bactéries ou des levures et moisissures connues pourraient être citées.

Le principe de l'invention consiste à préparer un échantillon de la matrice alimentaire, en ajoutant un agent fluidifiant à la matrice alimentaire prélevée. Cet agent fluidifiant doit présenter certaines caractéristiques :
- Il doit être ajouté dans une quantité adaptée pour fluidifier la matrice alimentaire, afin que le mélange obtenu puisse passer à travers le filtre d'un composant microfluidique destiné à la préparation de l'échantillon ;
- Il doit être ajouté dans une quantité adaptée pour éviter toute dégradation des espèces biologiques cibles, c'est-à-dire des bactéries cibles potentiellement présentes ; il peut notamment être présent dans une concentration comprise entre 5% et 25% du mélange final.
- Il doit permettre la fluidification de la matrice alimentaire, sans avoir à déployer de moyens trop importants et contraignants, permettant ainsi un déploiement facile sur le terrain ;
- Il doit être ajouté dans une quantité adaptée pour obtenir un mélange final de volume raisonnable, afin d'être engagé dans un composant microfluidique ;
- Il ne doit pas être un inhibiteur (persistant même après lavage) pour la réaction d'amplification moléculaire mise en œuvre postérieurement pour la détection ;

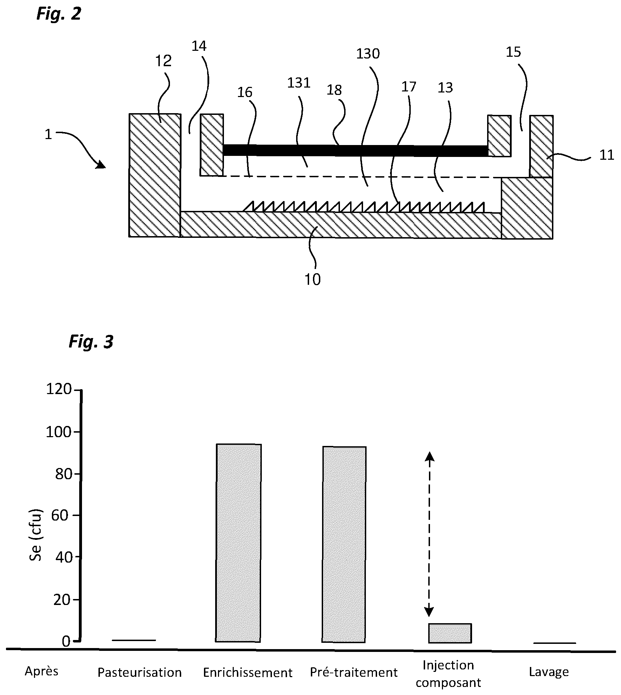

Comme indiqué ci-dessus, pour permettre au moins partiellement une préparation de l'échantillon et son analyse, on utilise avantageusement un composant micro-fluidique tel que représenté sur la figure 2.

Ce composant micro-fluidique 1 comporte un boîtier comprenant une paroi inférieure 10, une paroi latérale 11 et une paroi supérieure 12. Toutes les parois du boîtier seront réalisées dans un ou plusieurs matériaux. Ces matériaux seront par exemple aptes à pouvoir subir un chauffage dans une fourchette de température comprise entre 20°C et 100°C. Préférentiellement, certaines parois du boîtier seront réalisées dans un matériau transparent. Préférentiellement, le matériau employé sera un plastique, par exemple de type PMMA (Poly(Méthacrylate de Méthyle)) ou COC (Cyclic Olefin Copolymer).

Le composant 1 comporte une chambre 13 ménagée dans le boîtier. Cette chambre représente l'emplacement dans lequel peuvent être effectuées la purification/concentration et éventuellement la détection des espèces biologiques cibles (lorsque l'amplification est réalisée in situ). La chambre 13 est fermée vers le bas par la paroi inférieure du boîtier.

Le composant micro-fluidique comporte un premier canal 14 ménagé dans le boîtier et agencé pour injecter des fluides dans la chambre 13 ou pour évacuer des fluides en dehors de la chambre. Le premier canal 14 comporte une première extrémité comportant une ouverture ménagée par exemple à travers la paroi supérieure 12 du boîtier et une deuxième extrémité qui débouche dans ladite chambre 13. La première extrémité du premier canal 14 est par exemple agencée verticalement et sa deuxième extrémité débouche par exemple horizontalement dans la chambre 13. La première extrémité du premier canal est par exemple évasée pour y appliquer le cône d'une pipette ou sera adaptée au type de dispositif employé pour injecter le fluide dans le dispositif. A titre d'exemple, il peut s'agir d'une ouverture présentant un embout de type "luer" pour y connecter une seringue ou adaptée pour y connecter un circuit fluidique.

Le composant microfluidique comporte un deuxième canal 15 ménagé dans le boîtier. Ce deuxième canal 15 comporte également une première extrémité qui communique avec l'extérieur, formant une ouverture réalisée par exemple à travers la paroi supérieure du boîtier et une deuxième extrémité qui communique avec l'espace formé par la chambre 13. Par ce deuxième canal 15, on peut également injecter des fluides dans ladite chambre ou évacuer des fluides en dehors de ladite chambre. Sa première extrémité est par exemple agencée verticalement et sa deuxième extrémité horizontalement. La chambre 13 est placée entre le premier canal 14 et le deuxième canal 15. De manière identique, la première extrémité de ce deuxième canal est par exemple évasée pour y appliquer le cône d'une pipette ou sera adaptée au type de dispositif employé pour injecter le fluide dans le dispositif. A titre d'exemple, il peut s'agir d'une ouverture présentant un embout de type "luer" pour y connecter une seringue ou adaptée pour y connecter un circuit fluidique.

Vers le haut, la chambre 13 peut être fermée par une membrane 18 avantageusement souple et étirable, préférentiellement transparente. La paroi supérieure 12 du boîtier du dispositif comporte ainsi une ouverture qui est recouverte de manière hermétique par ladite membrane 18. Ladite membrane est ainsi ancrée dans le boîtier par toute solution de fixation adaptée, par exemple par collage. Cette membrane 18 sera par exemple composée d'un film, par exemple un film autocollant de type PET, d'épaisseur, de dimensions et de constitution adaptées pour se déformer de manière élastique, par rapport à ses points d'ancrage, notamment jusqu'au fond de la chambre 13.

Par le terme "transparent", on entend que le matériau employé est au moins partiellement transparent à la lumière visible, à la fluorescence ou à la luminescence, de manière à laisser passer au moins 80% de cette lumière. Il faut ainsi comprendre qu'il sera suffisamment transparent pour voir l'intérieur de la chambre 13, au moins le deuxième espace situé au-dessus du filtre 16 évoqué ci-dessous.

Le composant micro-fluidique 1 comporte un filtre 16 agencé dans ladite chambre 13, ce filtre 16 étant agencé pour séparer ladite chambre 13 en deux espaces. Les deux espaces sont par exemple superposés et désignés ainsi espace inférieur 130 situé sous le filtre et espace supérieur 131 situé au-dessus du filtre et sous la membrane 18. Ce filtre 16 est préférentiellement réalisé en tout ou partie sous la forme d'un film souple et fin, maintenu dans l'espace formé par la chambre de manière à ne permettre le passage d'un espace à l'autre que par les pores du filtre 16. Le film présente avantageusement une déformabilité élastique lui permettant de s'étirer lors de l'exercice d'une force d'appui dans une direction sensiblement verticale, cette déformabilité élastique ayant un niveau suffisant pour atteindre la paroi inférieure de la chambre 13. Le filtre 16 présente un diamètre moyen de pores compris entre 0.2 µm et 50 µm, par exemple compris entre 0.2 µm et 1 µm pour la séparation de microorganismes ou de 0.2 à 2µm pour retenir les bactéries.

Le diamètre des pores est bien entendu adapté pour assurer une séparation entre différentes espèces biologiques présentes dans l'échantillon. Le filtre 16 sera par exemple composé d'un film d'épaisseur, de dimensions et de constitution adaptée pour se déformer jusqu'au fond de la chambre 13 par rapport à ses points d'ancrage. Selon un mode de réalisation particulier, le filtre pourra être aussi réalisé dans un matériau transparent, par exemple avec les mêmes caractéristiques de transparence que la membrane.

L'un des traitements actifs pouvant être appliqué à l'échantillon peut consister en une lyse des espèces biologiques présentes dans l'échantillon. En cas de lyse, le composant micro-fluidique peut avantageusement comporter une surface d'appui rugueuse 17 agencée sur le fond de la chambre 13. Cette surface d'appui rugueuse 17 s'étend sur une partie majoritaire du fond de la chambre. Elle comporte un paramètre de rugosité de surface moyen compris entre 0.01µm et 10 µm, préférentiellement compris entre 0.2 µm et 3 µm. Cette surface d'appui rugueuse 17 est destinée à permettre une lyse mécanique des espèces biologiques présentes dans l'échantillon biologique placé dans le dispositif. Préférentiellement, la lyse mécanique est réalisée en broyant lesdites espèces biologiques, par abrasion sur ladite surface d'appui rugueuse. L'opération de broyage est mise en œuvre par un mouvement de friction des espèces biologiques contre la surface d'appui rugueuse, en employant un organe de broyage adapté. Cet organe sera par exemple une spatule ou une tige, par exemple en matériau plastique ou métallique. Cet organe est appliqué depuis l'extérieur de la chambre 13 et son extrémité est appliquée contre la surface externe de la membrane 18 de manière à étirer la membrane 18 et le filtre vers le fond de la chambre et ainsi frictionner les espèces biologiques présentes dans un échantillon contre la surface d'appui rugueuse 17.

Le composant microfluidique peut aussi être adapté pour permettre un traitement actif de type thermique de l'échantillon. Dans ce cas, le boîtier peut avantageusement intégrer des moyens de chauffage de l'espace interne de la chambre, composés par exemple d'au moins une résistance chauffante. La résistance est par exemple fixée sous la paroi inférieure du boîtier. Une source d'alimentation sera par exemple prévue pour alimenter la résistance. La source d'alimentation comportera par exemple une ou plusieurs piles électriques, fournissant suffisamment d'énergie pour chauffer la chambre à une température comprise dans la fourchette définie ci-dessus, c'est-à-dire de 20°C à 100°C. Bien entendu, d'autres moyens de chauffage pourraient être employés, comprenant par exemple une encre conductrice déposée par imprimerie ou sérigraphie sous la paroi inférieure du boîtier.

Ainsi, pour résumer, le composant micro-fluidique peut avantageusement comporter la structure "multicouches" suivante :
- Une surface inférieure d'appui rugueuse 17,
- Un espace inférieur 130 de la chambre 13, situé au-dessus de la surface d'appui rugueuse 17,
- Un filtre 16, avantageusement souple et étirable situé au-dessus de l'espace inférieur 130,
- Un espace supérieur 131 de la chambre 13 situé au-dessus du filtre 16,
- Une membrane 18, avantageusement souple et étirable située au-dessus de l'espace supérieur 131, fermant hermétiquement la chambre et accessible depuis l'extérieur du dispositif.

La paroi inférieure du composant et la membrane peuvent être réalisées dans des matériaux transparents, notamment pour mettre en œuvre une détection par amplification biomoléculaire, directement dans le composant.

De manière non limitative, le composant micro-fluidique peut présenter les caractéristiques dimensionnelles suivantes :
- Un premier canal 14 composé d'un canal d'entrée de 1mm de diamètre x 3mm de hauteur, puis d'un canal de section rectangulaire de 1mmx150µm de 3mm de long ;
- Une chambre 13 composée d'un espace inférieur 130 pour concentration/lyse qui présente un diamètre de 8mmx150µm de hauteur et d'un espace supérieur pour élution d'un diamètre 8mmx300µm de hauteur ;
- Filtre 16 de porosité adaptée à la cible à retenir (virus, levure, moisissure...), par exemple avec une porosité de 0.2 à 2 µm pour retenir les bactéries ;
- Un deuxième canal 15 composé d'un canal de section rectangulaire de 1mm x 150µm et de 3mm de long, puis d'un canal de 1mm de diamètre x3mm de hauteur ;

Il faut noter que le composant micro-fluidique présente notamment l'avantage d'optimiser la quantité d'espèces biologiques cibles obtenues et de les concentrer au maximum dans l'espace inférieur 130 de la chambre 13 du composant 1.

Dans le cadre du procédé de l'invention, une matrice alimentaire est initialement prélevée, cette matrice alimentaire pouvant être telle que définie ci-dessus, c'est-à-dire sous forme liquide ou légèrement pâteuse. Dans le cas d'une matrice alimentaire de type viande, le prélèvement sera liquéfié/dissout/désagrégé dans un liquide, et est ainsi sous forme liquide.

Le procédé de préparation de l'invention consiste initialement à ajouter à la matrice alimentaire prélevée, un agent fluidifiant formé d'un composé tensioactif de type Ethoxylate d'alcool secondaire. Ce type de composé est plus connu sous le nom commercial de Tergitol (marque déposée) et peut prendre différentes formulations. Il est notamment décrit dans la demande de brevet US2017/037339A1**.** De manière générale, il a la formule suivante :
R-O (CH₂CH₂O)ₙH dans laquelle R est une branche substituée ou non substituée par un groupe alkyle de type C₁₁₋₁₅, n correspond au degré d'éthoxylation compris entre 3 et 20.

De manière non limitative, le composé choisi pour la mise en œuvre du procédé de l'invention est choisi parmi le Tergitol 15-S-9 et le Tergitol TMN6. D'autres composés de la même famille pourraient cependant être envisagés.

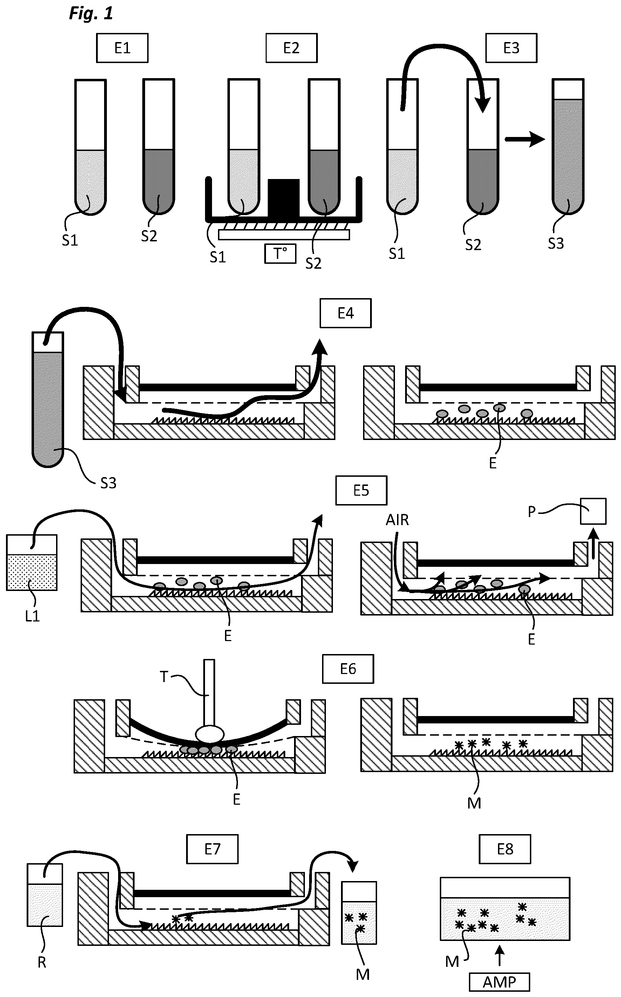

En référence à la figure 1, le procédé de l'invention suit les étapes suivantes :
E1 : On effectue un prélèvement de la matière alimentaire S1 à analyser. On prépare également une solution S2, par exemple à 20%, du composé tensioactif à ajouter audit prélèvement. L'échantillon S3 obtenu doit contenir au maximum 25% en concentration du composé tensioactif, avantageusement environ 10%. En effet, le volume de composé tensioactif ajouté doit être contrôlé pour éviter toutes dégradations des espèces biologiques cibles (les bactéries pathogènes) présentes dans le prélèvement de matrice alimentaire.
E2 : On vient chauffer (T°C) le prélèvement de matrice alimentaire S1 et la solution S2 de tensioactif. Le chauffage est par exemple réalisé à une température comprise entre 35°C et 50°C, avantageusement d'environ 45°C. Les deux solutions peuvent être chauffées séparément (comme sur la figure 1) ou être chauffées, après le mélange. La température de chauffage est choisie de manière à favoriser la fluidification de la matrice alimentaire, tout en évitant la dégradation des espèces biologiques cibles. Lorsque les deux solutions sont chauffées séparément, l'une pourrait être chauffée à une température supérieure à l'autre, dès l'instant que les conditions de préservation des espèces biologiques sont respectées après mélange.
E3 : Le composé tensioactif sélectionné est ajouté au prélèvement de matière alimentaire de manière à créer un échantillon S3 exploitable à engager dans un composant microfluidique 1 tel que décrit ci-dessus. L'échantillon S3 obtenu doit contenir au maximum 25% en concentration du composé tensioactif, avantageusement environ 10%. Comme indiqué ci-dessus, le volume de composé tensioactif ajouté, la température et la durée du traitement doivent être contrôlés pour éviter toutes dégradations des espèces biologiques E cibles (les bactéries pathogènes) présentes dans la matrice alimentaire.

A partir de l'étape E4 décrite ci-dessous, l'échantillon S3 obtenu après mélange est avantageusement traité en utilisant le composant microfluidique 1 décrit ci-dessus.

E4 : L'échantillon S3 préparé est injecté à travers le premier canal 14 du composant microfluidique. Le fluide passe à travers le filtre 16 et est évacué par le deuxième canal 15, tandis que les espèces biologiques E cibles restent dans l'espace inférieur 130 de la chambre.

E5 : Un lavage des espèces biologiques E cibles retenus dans l'espace inférieur 130 est réalisé avec un tampon de lavage L1. Puis un séchage est avantageusement opéré par injection d'air (AIR) à travers le circuit microfluidique du composant 1, via le premier canal 14. Une pompe P peut être placée en aval pour accélérer le séchage.

E6 : On effectue une lyse des espèces biologiques présentes dans l'espace inférieur de la chambre en venant les broyer contre la surface inférieure 17 rugueuse, pour libérer les molécules M d'ADN. On utilise par exemple une tige T ou spatule venant exercer un appui contre la membrane 18.

E7 : On effectue une élution des molécules d'ADN à travers le filtre 16 du composant, par exemple en utilisant un tampon d'élution, par exemple le réactif d'amplification R nécessaire pour la réaction d'amplification qui va suivre.

E8 : On effectue une réaction d'amplification AMP, par exemple de type PCR, LAMP, pour identifier la présence des bactéries pathogènes dans la matrice alimentaire.

En variante de réalisation, il serait également possible, après l'étape E4 décrite ci-dessus, de réaliser un lavage des espèces biologiques E cibles retenues dans l'espace inférieur 130 de la chambre 13, suivi de l'injection d'un milieu de culture. Ensuite, on vient fermer le composant microfluidique en obturant ces deux canaux. On vient favoriser la croissance bactérienne par incubation (par exemple par chauffage à 37°C pendant 2h).

Une fois la période d'incubation terminée, on effectue un lavage des espèces biologiques E présentes dans l'espace inférieur 130, puis un séchage. Les étapes E7 et E8 décrites ci-dessus sont ensuite mises en œuvre de manière identique.

De manière plus simple, selon une autre variante de réalisation, après l'étape E4, on effectue un lavage, puis une élution des espèces biologiques E retenues dans l'espace inférieur 130 de la chambre 13. On effectue ensuite une analyse, selon différentes méthodes (analyse en culture, visualisation microscope, antibiogramme, immunologie...).

De manière non limitative, pour une matrice alimentaire telle que du lait, le procédé peut être mise en œuvre selon les conditions suivantes :
- Préparation des solutions :
   La solution de tensioactif est à base de Tergitol 15S9, à 20%.

Le tampon de lavage utilisé est par exemple :
- Tris HCl pH8 10mM
- SALMON DNA 5 mg/mL
- BSA 0,5%

- Le chauffage est réalisé séparément pour la solution de Tergitol et le lait, à une température de 45°C sans agitation pendant 5 min ;
- On utilise un préfiltre, en amont du filtre 16 présent sur le composant microfluidique ;
- Un rinçage des espèces biologiques peut être réalisé avec 1mL solution de Ringer ;
- Le lavage est réalisé avec 3mL du tampon de lavage ;
- Le séchage est réalisé avec une seringue vide, en injectant 5mL d'air sur le composant pendant qu'une pompe branchée sur le deuxième canal 15 crée une aspiration ;
- La lyse mécanique est réalisée manuellement ;
- On réalise l'élution avec le tampon d'élution du kit de détection utilisé ;

La figure 3 montre un diagramme de suivi de la présence des bactéries dans une matrice alimentaire de type lait, lors de sa préparation et des différentes étapes d'analyse.

De ce diagramme, on comprend que :
- Lorsque le lait est pasteurisé, il n'y a plus de bactéries (Pasteurisation) ;
- Ajout de la bactérie « Salmonella » au lait (Enrichissement) ; toutes les bactéries sont donc présentes ;
- Le prétraitement du lait constitué du chauffage séparé du composé S2 (Tergitol 20%) et du lait à 45°C + mélange des deux, n'entraîne aucune perte de bactéries Salmonella donc le procédé fluidifie le lait sans endommager les bactéries ;

- Les bactéries sont retenues sur le filtre lors de l'injection dans le composant, donc le procédé ne détériore pas le filtre du composant qui reste intact et capture la majorité des bactéries ;
- Après le lavage, aucune bactérie n'est éluée, donc les bactéries sont bloquées sur le filtre ;

Le principe de l'invention a également été appliqué, avec réussite, sur des échantillons non enrichis de steak haché à 15% de matière grasse (broyé dans EPT ou du ringer après dilution au 1/5).

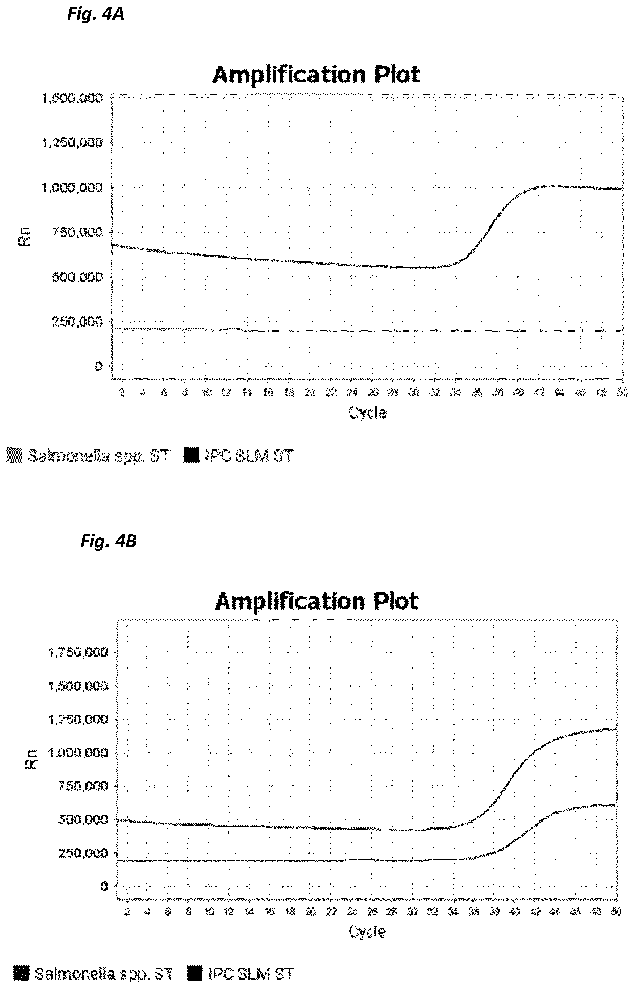

Les figures 4A à 4C montrent des diagrammes de résultats pour une matrice alimentaire de type steak haché.

On utilise ainsi deux prélèvements distincts :
- Premier prélèvement : steak haché 15%MG, J-4 de la DLC (date limite de consommation), contaminé en salmonelle ;
- Deuxième prélèvement : steak haché 15%MG, J-4 de la DLC non contaminé en salmonelle ;

On réalise ensuite trois échantillons distincts par dilution de la matrice alimentaire : 25g de steak haché, dans 100g de liquide. Dilution au 1/5 dans de l'eau peptonnée tamponée (EPT) ou au 1/5 dans « ringer » dans un sac pour malaxeur.
- Echantillon A : 25g steak haché 15%MG, J-4 de la DLC non contaminé en salmonelle + 100g ringer
- Echantillon B :25g steak haché 15%MG, J-4 de la DLC contaminé en salmonelle + 100g ringer
- Echantillon C : 25g steak haché 15%MG, J-4 de la DLC non contaminé en salmonelle + 100g EPT

Chaque échantillon A, B et C est passé au broyeur/malaxeur/homogénéisateur pendant 1 minute.

On effectue un prélèvement sur chaque échantillon A, B et C. Le processus décrit ci-dessus aux étapes E2 à E8 est ensuite mis en œuvre.

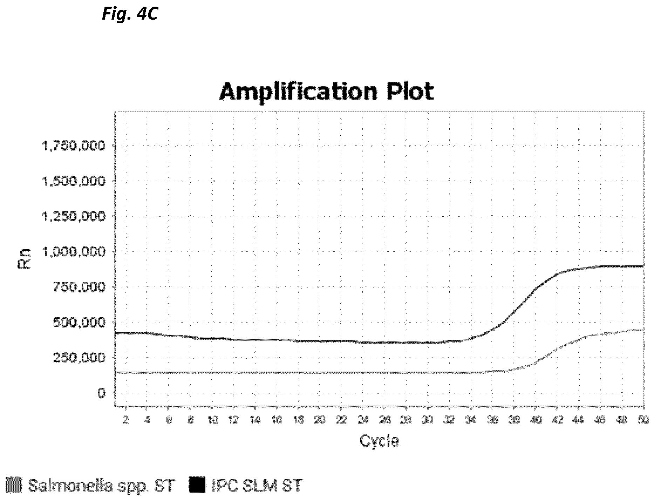

Les diagrammes de la figure 4A, figure 4B et de la figure 4C sont des courbes d'amplification qPCR, avec mesure de la fluorescence (brute, Rn) en fonction du cycle de réaction pour un contrôle interne positif et salmonella.

Sur les trois diagrammes, on constate que le contrôle interne positif est détecté dans les trois essais, indiquant l'absence d'inhibiteur de réaction.

De plus, on constate que la salmonelle n'est détectée que dans les échantillons de steak haché contaminé en salmonelle. La solution de l'invention est donc parfaitement fiable et robuste.

L'invention présente ainsi de nombreux avantages, parmi lesquels :
- La possibilité de traiter divers types de matrice alimentaire, sans enrichissement, permettant ainsi un gain de temps significatif ;
- La possibilité d'engager un volume d'échantillon plus important à l'intérieur du composant, et donc de fiabiliser la détection des bactéries dans la matrice prélevée ;
- Un procédé simple, fiable et robuste, facilement déployable sur le terrain, notamment car il est rapide et qu'il utilise du matériel facilement transportable ;

## Revendications

1. Procédé de préparation d'un échantillon (S3) de matrice alimentaire, **caractérisé en ce qu'**il consiste à réaliser un mélange d'un prélèvement (S1) de ladite matrice alimentaire avec une solution (S2) comprenant un composé tensioactif de type Ethoxylate d'alcool secondaire, ledit procédé comportant également une étape de chauffage dudit échantillon (S3) obtenu, ou de chauffage préalable avant mélange de la solution (S2) contenant ledit composé tensioactif et du prélèvement (S1) de ladite matrice alimentaire, à une température adaptée pour éviter toute dégradation d'espèces biologiques (E) cibles dans l'échantillon, ledit composé tensioactif étant ajouté au prélèvement (S1) de matrice alimentaire de manière à ne pas dépasser 25% en concentration dans l'échantillon (S3) obtenu, ledit procédé étant **caractérisé en ce qu'**il comporte une étape de filtration dudit échantillon (S3) obtenu, mise en œuvre à travers un filtre (16) en vue d'isoler lesdites espèces biologiques (E) cibles.

2. Procédé selon la revendication 1, **caractérisé en ce que** le prélèvement (S1) de matrice alimentaire et la solution (S2) contenant le composé tensioactif sont chauffés séparément à une température comprise entre 35°C et 50°C.

3. Procédé selon la revendication 1, **caractérisée en ce qu'**une étape de lyse desdites espèces biologiques (E) isolées est mise en œuvre en vue de libérer les molécules d'ADN.

4. Procédé selon la revendication 3, **caractérisée en ce qu'**une étape d'élution des molécules d'ADN libérées à l'aide d'un tampon d'élution est mise en œuvre après ladite état de lyse.

5. Procédé selon la revendication 4, **caractérisée en ce que** le tampon d'élution est un réactif d'amplification (R).
